# EUROPEAN PATENT SPECIFICATION

(11) **EP 2 563 148 B1**
(45) Date of publication and mention of the grant of the patent: **24.08.2016**
(21) Application number: 11727815.0
(22) Date of filing: 23.04.2011
(51) Int. Cl.: A01N 59/16, A61L 12/06, A61L 12/08, A01N 59/00, A01P 1/00, A01N 43/08, A01N 43/16, A01N 31/16, A01N 37/40

(54) **COMPOSITION IN THE FORM OF LIQUID FOR MAINTENANCE OF CONTACT LENSES AND MEDICAL MATERIALS**
FLÜSSIGE ZUSAMMENSETZUNG ZUR BEHANDLUNG VON KONTAKTLINSEN UND MEDIZINISCHEN MATERIALIEN
COMPOSITION SOUS FORME DE LIQUIDE POUR L'ENTRETIEN DE LENTILLES DE CONTACT ET DE MATÉRIAUX MÉDICAUX

(30) Priority: 26.04.2010 PL 39105610
(43) Date of publication of application: 06.03.2013
(73) Proprietor: Uniwersytet Jagiellonski, 31-007 Krakow (PL)
(72) Inventor: LABUZ, Przemyslaw, PL-31-636 Kraków (PL); MACYK, Wojciech, PL-32-060 Liszki (PL); STOCHEL, Grazyna, PL-31-235 Kraków (PL); HECZKO, Piotr B., PL-31-517 Kraków (PL); STRUS, Magdalena, PL-30-617 Kraków (PL)
(74) Representative: Witek, Rafal
(86) International application number: PCT/PL2011/050011
(87) International publication number: WO 2011/136672

(56) References cited:
- WO-A1-2008/019869
- WO-A2-2010/098687
- RAJH T ET AL: "Surface Restructuring of Nanoparticles: An Efficient Route for Ligend-Metal Oxide Grosstalk", JOURNAL OF PHYSICAL CHEMISTRY. B (ONLINE), AMERICAN CHEMICAL SOCIETY, COLUMBUS, OH, US, vol. 106, no. 41, 1 January 2002 (2002-01-01), pages 10543-10552, XP003013547, ISSN: 1520-5207, DOI: 10.1021/JP021235V
- RAJH: "Improving optical and charge separation properties of nanocrystalline TiO2 by surface modification with vitamin C", THE JOURNAL OF PHYSICAL CHEMISTRY B, vol. 103, no. 18, 1 January 1999 (1999-01-01), pages 3515-3519, XP55001506, ISSN: 1520-6106
- L. DE LA GARZA: "Surface states of titanium dioxide nanoparticles modified with enediol ligands", THE JOURNAL OF PHYSICAL CHEMISTRY B, vol. 110, no. 2, 1 January 2006 (2006-01-01), pages 680-686, XP55001508, ISSN: 1520-6106
- BENJAMIN CHOQUENET ET AL: "Quercetin and Rutin as Potential Sunscreen Agents: Determination of Efficacy by an in Vitro Method", JOURNAL OF NATURAL PRODUCTS, vol. 71, no. 6, 1 June 2008 (2008-06-01), pages 1117-1118, XP55008425, ISSN: 0163-3864, DOI: 10.1021/np7007297
- DATABASE WPI Week 200829 Thomson Scientific, London, GB; AN 2008-E02415 XP002660257, & CN 101 028 528 A (QUAN L) 5 September 2007 (2007-09-05)
- DATABASE WPI Week 200362 Thomson Scientific, London, GB; AN 2003-649024 XP002660258, & JP 2003 093481 A (MENICON CO LTD) 2 April 2003 (2003-04-02)

## Description

The subject of the present invention is a composition, especially in the form of a liquid for an external use, for treatment and maintenance of contact lenses, containing nanocrystalline photocatalyst active upon visible light illumination and hydrogen peroxide.

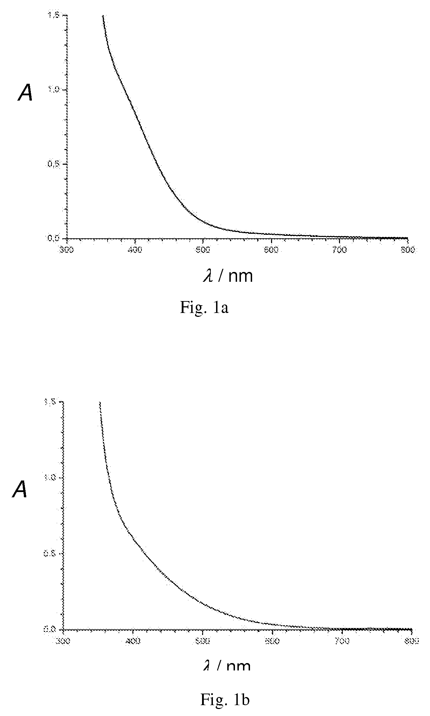

WO2010/098687, published after the priority date of the present invention, discloses compositions with strong photocatalytic properties for sterilizing various materials comprising the surface modified TiO₂ particles similar to the described in subject Figures 1a - 1c, 2, 3a-3g and 4 as well as Examples 1 - 4 of the present application also have been disclosed in this citation.

Another compositions comprising surface-modified TiO₂ with different organic compounds have been disclosed also by Rajh et al. (Journal of Physical Chemistry, vol. 106, no. 41, 2002; Journal of Physical Chemistry, vol. 103, no. 18, 1999) and L. De la Garza (Journal of Physical Chemistry, vol. 110, no. 2, 2006).

Moreover, JP 2003 093 481 A and WO2008/019869 disclose disinfectant compositions comprising photodynamic nanoparticles of TiO₂ in combination with hydrogen peroxide.

However, the state of the art does not suggest surprising stability at pH=7 of colloids obtained from surface-modified TiO₂ with selected compounds as it has been disclosed in the subject invention.

The goal of the present invention is to deliver a composition for the production of solutions exhibiting sterilizing properties upon irradiation with visible light and therefore suitable for maintenance of various materials, especially those requiring physiological use, as contact lenses, medical materials (catheters, bandages, syringes, etc.).

Unexpectedly, such a defined goal has been achieved by the present invention.

The subject of the present invention is the composition in the form of liquid containing colloidal solution, with surface-modified titanium dioxide nanoparticles smaller than 100 nm as a dispersed phase, and the solution of hydrogen peroxide as the dispersing medium, wherein said titanium dioxide nanoparticles are surface-modified with an organic compound, characterized in that it exhibits stability in aqueous solution with a pH of about 7, and the organic compound is a compound selected from the group consisting of: disodium salt of 4,5-dihydroxybenzene-1,3-disulfonic acid, rutin or ascorbic acid.

Preferably, the composition according to the present invention exhibits visible light absorption in the wavelength range (λ) of not less than approximately 400 nm to about 600 nm, preferably up to about 700 nm, and ultraviolet light absorption (λ < 400 nm).

Preferably, the composition according to the present invention additionally contains a buffer system, preferably isotonic, to maintain the pH in a pharmaceutically acceptable range.

Preferably, the composition according to the present invention contains surface-modified nanocrystalline titanium dioxide within the concentration range of 0.02 g dm to 1 g dm⁻³, hydrogen peroxide, preferably in the amount within the range of 0.001 g dm⁻³ to 0.1 g dm⁻³, and a stabilizer of hydrogen peroxide, preferably EDTA.

Preferably, the composition according to the present invention contains additionally an additive showing bacteriocidal and/or mycocidal activity.

The composition according to the invention can additionally contain pharmaceutical carriers and excipients. As carriers and excipients the known carriers and excipients used in pharmacy, that are compatible with the active substance, can be applied.

In an exemplary composition the modified titanium dioxide characterized by particle size not exceeding 100 nm constitutes stable colloidal systems in solution of PBS buffer, retaining its photocatalytic activity. Preferably, the composition according to the invention constitutes a clear, transparent aqueous solution, containing hydrogen peroxide and nanocrystalline titanium dioxide, the surface of which has been modified with rutin, as the active substances, together with NaCl and phosphate buffer.

Preferably, the composition according to the invention or the colloidal solution of titanium dioxide nanoparticles is prepared as a concentrate, preferably containing 40-50 times higher concentration of titanium dioxide as compared to its final concentration in the composition. To obtain the final composition, the concentrate should be diluted with water, preferably with aqueous buffer solution, isotonic aqueous solution, hydrogen peroxide and eventually other additives to the final concentration of all ingredients.

Preferably, the composition according to the invention is used to the external use as a contact lens cleaning solution. In general, the composition can be used in the production of a preparation for sterilisation or disinfection, preferably in the production of a photosterilising, photobacteriocidal, photomycocidal, or photocatalytic preparation particularly designed for use in one of the following areas: cosmetology, dermatology, ophthalmology, laryngology, urology, gynaecology, rheumatology, oncology, surgery, veterinary medicine, dentistry, in particular for the sterilization of surfaces and glass or plastic elements, particularly contact lenses, medical catheters, glass and/or plastic conduits as well as other surfaces, the sterilization of which is desirable and/or required.

The composition according to the present invention exhibits photocatalytic activity upon irradiation with visible light λ > 400 nm; photocatalysis is the result of the absorption of visible light by the titanium surface complex of the *charge*-*transfer* type), as well as ultraviolet light (λ < 400 nm; photocatalysis is the result of the absorption of ultraviolet light by the titanium surface complex of the *charge*-*transfer* type or directly by titanium dioxide). The irradiation generates so-called reactive oxygen species (OH^{•}, O₂^{•-}, H₂O₂, ¹O₂).

Unexpectedly it has been shown, that addition of small amounts of peroxides, e.g. hydrogen peroxide, intensifies the photocatalytic properties of the photocatalyst described above. The electron transfer from the conduction band of titanium dioxide to the peroxide molecule leads to the formation of highly oxidative hydroxyl radicals as a consequence of the peroxide reduction. In the presence of trace amounts of metal ions a catalytic decomposition of H₂O₂ to OH^{•} may occur; the reaction known as a Fenton process. This process unfavorably influences the stability and durability of the composition, however it can be inhibited by addition of agents complexing metal ions, e.g. EDTA (ethylenediaminetetraacetic acid or its salt), in amounts assuring binding of all transition metal ions present in the composition.

Example embodiments of the present invention have been shown in figures, in which Fig. 1 shows UV-vis absorption spectra of a colloidal solution of TiO₂ nanocrystals modified respectively with compound K-1 (Table 2) (a), ascorbic acid (b) and rutin (c) (each concentration: 0.4 g dm⁻³); Fig. 2 shows a TEM image recorded for the material K-1@TiO₂; Fig. 3 shows the results of irradiation with visible light (using cut-off filters) of a reaction mixture (bovine albumin with a solution of colloidal TiO₂ nanocrystals modified respectively with compound K-1, ascorbic acid and rutin), in the electrophoretic image: K-1@TiO₂: (a) λ > 400 nm, (b) λ > 435 nm, (c) λ > 455 nm, KA@TiO₂: (d) λ > 400 nm, (e) λ > 420 nm, rutin@TiO₂: (f) λ > 420 nm, (g) λ > 455 nm; Fig. 4 shows E. *coli* viability tests in the presence of a colloidal solution of TiO₂ nanocrystals modified with K-1 (grey) and in the absence of the photocatalyst (black) (a) during irradiation with light λ > 420 nm and (b) *E*. *coli* viability test results in the same system in the dark, as well as *E. coli* viability tests in the presence of a colloidal solution of TiO₂ nanocrystals modified with ascorbic acid (squares) and in the absence of the photocatalyst (triangles) (c) upon irradiation with light λ > 420 nm and (d) *E. coli* viability test results in the same system in the dark. Figure 5 shows the results described in the Example 5 of the comparative tests of photoactivity of the composition according to the present invention, or its components, in the process of proteins oxidation. Figure 6 shows the results described in the Example 6 of the comparative tests of photoactivity of the composition according to the present invention, or its components, in the process of bacteria inactivation. Figure 7 presents the results of azure B photooxidation proving the unexpected synergy between activity of the photocatalyst and hydrogen peroxide.

### Example 1. Production of a nanocrystalline photocatalyst active in visible light

The initial substrate for the synthesis of the materials in question is an unmodified nanocrystalline TiO₂, which may be produced according to various known procedures. One of them is proposed by Wang et al. (J. Phys. Chem. B, 2000, 104, 93-104). Particles of titanium dioxide TiO₂ smaller than 100 nm (estimated using imaging with a transmission electron microscope) is modified on its surface directly via chemisorption of an organic compound selected from the group according to the present invention with the formation of *charge-transfer* complexes. Photoinduced electron transfer occurs between the organic compound molecule and the semiconductor particle. A photoactive colloid according to the present invention is characterized by a high degree of dispersion and occurs in the form of a suspension or emulsion.

**Variant 1.** A colloidal aqueous solution of TiO₂ (1.2 g dm⁻³) containing isopropanol (10%) in a nitric acid (HNO₃) environment (pH = 2.5) was supplemented with crystalline 4,5-dihydroxy-1,3-benzenedisulfonic acid disodium salt (K-1; Table 2) at a molar ratio of 1:1 (modifier:TiO₂). A yellow precipitate was formed. The resulting suspension was alkalized with an NaOH solution to pH = 7 (causing the precipitate to dissolve). The solution was placed in a dialysis tube and dialyzed twice against water or an appropriate buffer (i.e. SSC or PBS) in order to remove alcohol and the excess of modifier not bound with TiO₂. A yellow, clear colloidal solution was produced, which was used in further experiments.

The method described is equally suitable for synthesizing nanocrystalline TiO₂ modified with catechol derivatives or salicylic acid or phthalic acid derivatives (syntheses 2 and 3, Tables 1 and 2). In these cases it proved impossible to obtain stable materials (ones that do not undergo aggregation) at pH ≈ 7.

**Variant 2.** A colloidal aqueous solution of TiO₂ (1.2 g dm⁻³) containing isopropanol (10%) in a nitric acid environment (HNO₃, pH = 2.5) was supplemented with a compound from the group A (A-1:A-2; phthalic acid derivatives) or S (S-1:S-7; salicylic acid derivatives; Table 1) in crystalline form, at a molar ratio of 1:1 (modifier:TiO₂). The colloidal solution changed its color. The resulting colloidal solution was alkalized with an NaOH solution to pH = 7. The solution was placed in a dialysis tube and dialyzed twice against water, in order to remove alcohol and any modifier not bound with TiO₂.

**Table 1. Phthalic acid and salicylic acid derivatives.**

| **Compound symbol** | **Compound name** | **Structural formula** |
|---|---|---|
| A-1 | phthalic acid | 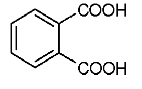 |
| A-2 | 4-sulfophthalic acid | 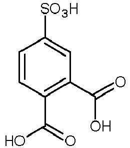 |
| S-1 | 4-amino-2-hydroxybenzoic acid | 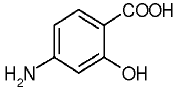 |
| S-2 | 3-hydroxy-2-naphthylic acid | 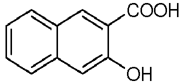 |
| S-3 | salicylic acid | 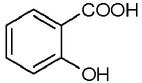 |
| S-4 | 6-hydroxysalicylic acid | 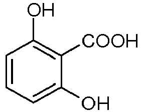 |
| S-5 | 5-hydroxysalicylic acid | 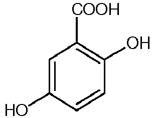 |
| S-6 | 5-sulfosalicylic acid | 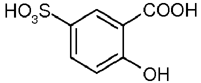 |
| S-7 | 3,5-dinitrosalicylic acid | 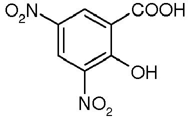 |

**Variant 3.** A colloidal aqueous solution of TiO₂ (1.2 g dm⁻³) containing isopropanol (10%) in a nitric acid environment (HNO₃, pH = 2.5) was supplemented with a compound from the group K (K-2:K-8; Table 2) at a molar ratio of 1:1 (modifier:TiO₂). The colloidal solution changed its color. The resulting colloidal solution was alkalized with an NaOH solution to pH = 7. The solution was placed in a dialysis tube and dialyzed twice against water, in order to remove alcohol and any modifier not bound with TiO₂.

**Table 2. Catechol derivatives.**

| **Compound symbol** | **Compound name** | **Structural formula** |
|---|---|---|
| K-1 | disodium salt of 1,4-dihydroxy-1,3-benzenedisulfonic acid | 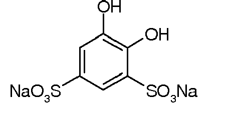 |
| K-2 | gallic acid | 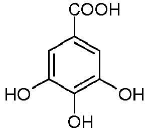 |
| K-3 | pyrogallol | 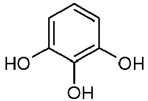 |
| K-4 | 2,3-naphthalenediol | 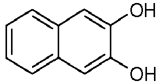 |
| K-5 | 4-methylcatechol | 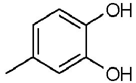 |
| K-6 | 3,5-di-*tert*-butylcatechol | 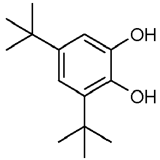 |
| K-7 | *p*-nitrocatechol | 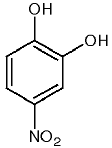 |
| K-8 | 3,4-dihydroxy-L-phenylalanine (DOPA) | 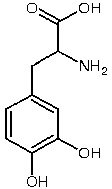 |

**Variant 4A.** A colloidal aqueous solution of TiO₂ (1.2 g dm⁻³) containing isopropanol (10%) in a nitric acid environment (HNO₃, pH = 2.5) was supplemented with a compound with the formula (rutin): 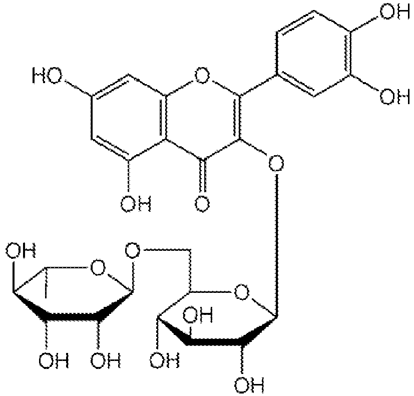 at a molar ratio of 1:1 (rutin:TiO₂). An orange precipitate was formed. The resulting suspension was alkalized with an NaOH solution to pH = 9 (causing the precipitate to dissolve). The solution was placed in a dialysis tube and dialyzed three times. The first dialysis was performed against an aqueous solution of NaOH, pH = 9. The subsequent two dialyses were performed against distilled water, or appropriate buffer (i.e. SSC or PBS, pH ≈ 7) in order to remove alcohol and any modifier not bound with TiO₂. An orange, clear colloidal solution was obtained which was used in subsequent experiments.

**Variant 4B.** A colloidal aqueous solution of TiO₂ (1.2 g dm⁻³) containing isopropanol (10%) in a nitric acid environment (HNO₃, pH = 2.5) was supplemented with a crystalline compound with the formula (rutin): 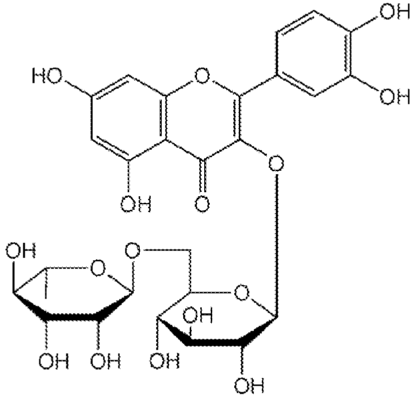 at a molar ratio of 1:1 (rutin:TiO₂). An orange precipitate appeared. The resulting suspension was centrifuged. The precipitate was rinsed several times with an aqueous HCl solution (pH = 2 to 4) until excess modifier (rutin) was rinsed out, which was confirmed spectrophotometrically. Next, the precipitate was suspended in water or a buffered aqueous solution. A clear, stable colloidal solution was produced.

**Variant 5.** A colloidal aqueous solution of TiO₂ (1.2 g dm⁻³) containing isopropanol (10%) in a nitric acid environment (HNO₃, pH = 2.5) was supplemented with a crystalline ascorbic acid (KA) in a molar ratio 1:1 (KA:TiO₂); appearance of orange color was observed. Then, the resulting sol was adjusted to pH = 7 and placed in a dialysis bag and dialyzed twice against an aqueous solution of ascorbic acid (5 mmol dm⁻³, pH = 7). Orange, clear, colloidal solution was obtained, which was used in further experiments. Nanocrystals of TiO₂ modified with ascorbic acid (KA@TiO₂) were stable (did not undergo aggregation) at pH = 7.

TiO₂ nanocrystals modified with ascorbic acid or another organic compound according to the present invention may then be subjected to further modifications consisting of conjugation with a molecule increasing the specificity of their activity (i.e. an antibody, peptide, biotin or vitamins).

### Example 2 Characteristics of the synthesized materials

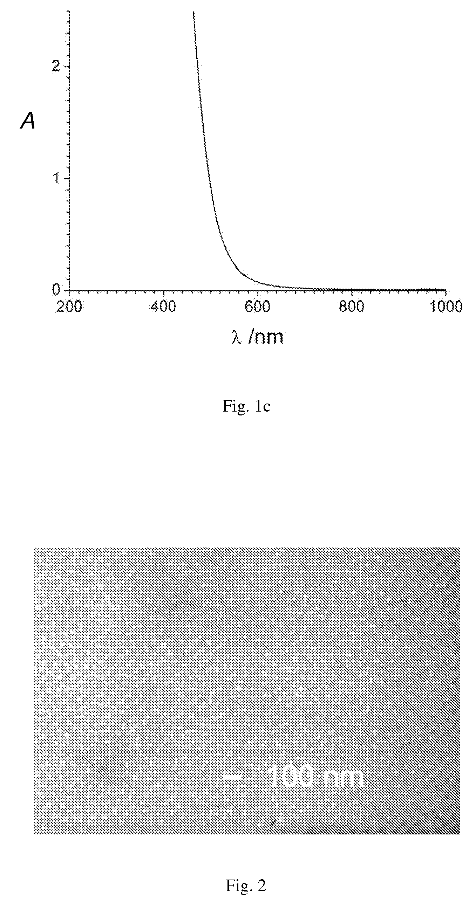

Within the group of materials synthesized as described in Example 1 TiO₂ nanocrystals modified by modifier K-1 (K-1 @ TiO₂), rutin (rutin@TiO₂) and ascorbic acid (KA @ TiO₂) showed stability at pH = 7. Other materials underwent aggregation, which was manifested in the precipitation at pH > 3-4. UV-vis spectrum of the K-1 @ TiO₂ is shown in Fig 1a. Like other materials (stable in acidic solutions) it shows absorption of visible light up to ca. 500-700 nm. The transmission electron microscope image presented in Figure 2 confirms the homogeneity of the material - separated particles, showing no tendency to aggregate are several to twenty nanometers in size. UV-vis spectrum of the KA @ TiO₂ material is shown in Figure 1b. Like other materials (stable in acidic solutions) KA @ TiO₂ shows absorption of visible light up to ca. 600-700 nm. UV-vis spectrum of the rutin @ TiO₂ solution is shown in Fig 1c. The material shows absorption of visible light to a wavelength of 600 nm.

### Example 3. Photocatalytic activity of the materials

Photodegradation tests were carried out with bovine serum albumin (as a model protein) upon visible light irradiation in the presence of nanocrystalline TiO₂ modified by organic compound selected from the group according to the present invention. The protein concentration in solution was monitored using semi-quantitative method of polyacrylamide gel electrophoresis under denaturing conditions (SDS-PAGE). The reaction mixtures, irrespective of the modification of nanocrystalline TiO₂, were prepared as follows:

A solution of modified TiO₂ (in an amount to yield a final concentration of 0.4 mg/ml in terms of TiO₂) was mixed with bovine albumin (final concentration 0.4 mg/ml) and water to a final volume of 2 ml.

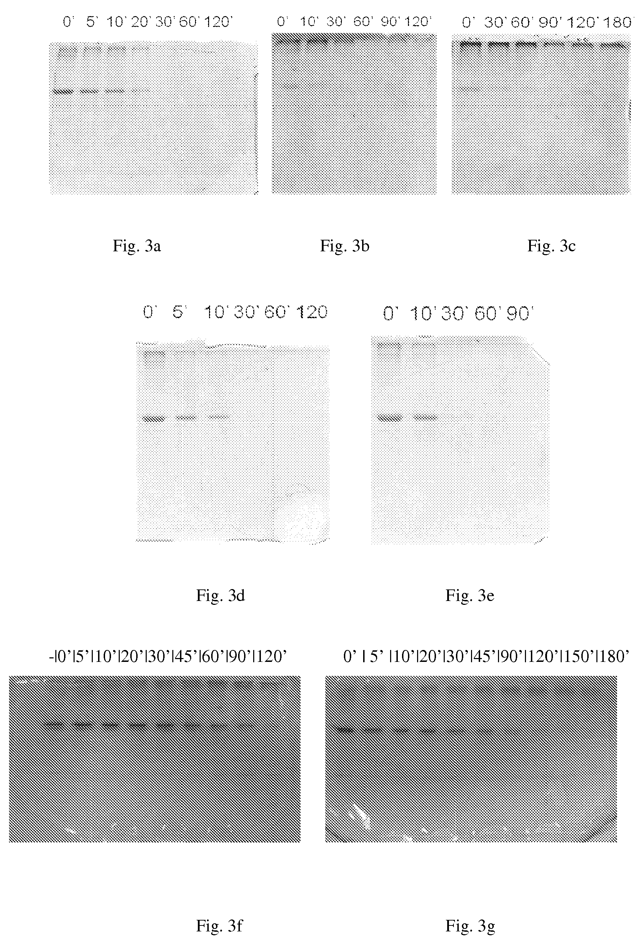

Irradiation was performed using a high-pressure mercury lamp, HBO-500, as a light source and filters delivering light at a wavelength in the range 420-800 nm. Tests were performed in a quvette purged with a small stream of air during irradiation to ensure a constant oxygen level in solution. During the experiment, samples were taken and subjected to electrophoresis following denaturation in order to monitor the degree of albumin degradation. The electrophoresis was performed in a Laemmli system using a 10% separating gel and a 4% stacking gel. The results are shown in Fig. 3. Sample 0' corresponds to an image of 2 µg of protein.

The photodegradation assays of protein have confirmed the high level of photocatalytic activity of K-1 @ TiO₂ upon visible light irradiation. The compiled protein electrophoresis images show protein degradation over the exposure time in a sample containing the protein and modified titanium dioxide in water. In the spectral range of 400-800 nm (Fig. 3a) one can observe a clear decrease of protein concentration during irradiation. In a narrower radiation ranges, 435-800 nm and 455-800 nm, the results are slightly poorer than the initial ones (Fig. 3b and 3c).

Protein photodegradation assays have confirmed the high level of photocatalytic activity of KA @ TiO₂ upon visible light irradiation. During irradiation within the spectral ranges 400-800 nm and 420-800 nm (Fig. 3d and 3e, respectively) a clear decrease in protein concentration can be observed within several minutes.

Protein photodegradation assays have confirmed the high level of photocatalytic activity of rutin @ TiO₂ upon visible light irradiation. The compiled protein electrophoresis images show protein degradation over the exposure time in a sample containing the protein and modified titanium dioxide in water. Both in the spectrum range 420-800 nm (Fig. 3f) and in a narrower range of radiation, 455-800 nm (Fig. 3g) one can observe clear decrease in protein concentration throughout the time of exposure.

### Example 4. Evaluation of the activity of the material in the photoinactivation of bacteria

The assays of the ability of the new materials to photoinactivate microorganisms were performed on a model strain of *Escherichia coli,* and the absence of cytotoxicity in this material to this bacterial strain was demonstrated. The tests assaying the efficiency of the photoinactivation of microorganisms were performed thusly:

A suspension of bacteria in water (ca. 10⁶ CFU/ml determined spectrophotometrically) was supplemented with a colloidal solution of nanocrystalline TiO₂ modified with an organic compound selected from the group according to the present invention, to its final concentration of 0.4 mg/ml. The assays were performed under the same irradiation conditions as the protein photodegradation assays (2 ml samples, see above) using a cut-off filter *λ* > 420 nm, aerating the sample during irradiation. Small samples were collected in order to evaluate the colony-forming potential of the examined strain. The colony formation ability was determined in dishes through the inoculation of 100 µl aliquots of a serial dilution of the bacterial suspension onto minimal medium (DIFCO) and counting the colonies. The results are shown as cell survival fractions *S*/*S*₀.

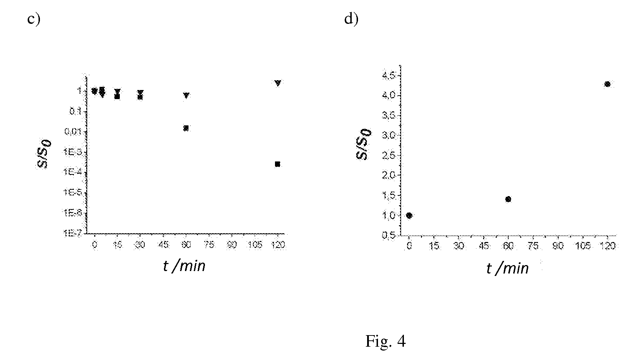

The results obtained for K-1 @ TiO₂ demonstrate its high activity in the photoinactivation of microorganisms, using the model strain of *E. coli* (Fig. 4a). At the same time, the cytotoxicity of the tested materials was not observed against this strain (Fig. 4b). Analogous results were obtained for KA @ TiO₂ (photoinactivation - Fig. 4c and cytotoxicity - Fig. 4d).

### Example 5 . Comparison of activity of the composition according to the invention and composition without the additives active towards proteins

Photodegradation tests were carried out with bovine serum albumin (a protein as a model substrate) under visible light irradiation in the presence of the composition based on nanocrystalline TiO₂ modified with rutin. The protein concentration in solution was monitored using semi-quantitative method of polyacrylamide gel electrophoresis under denaturing conditions (SDS-PAGE). The reaction mixtures, irrespective of the modification of nanocrystalline TiO₂, were prepared as follows:

The solution of modified TiO₂ (final concentration of 0.32 g dm⁻³ in terms of TiO₂), bovine serum albumin (final concentration of 0.2 g dm⁻³) and water were mixed together to a final volume of 2 ml.

Tests were performed in chambers of 2 ml capacity using LEDs as the light source (λₘₐₓ = 470 nm, power of 30 mW). During the experiment, collected samples after protein denaturation were subjected to electrophoretic analysis to determine the extent of albumin degradation. Electrophoresis was performed using Laemni setup with 10% separating gel and 4% stacking gel. The results of measurements are presented in the following figures (sample 0' corresponds to the image of 1 µg of protein):

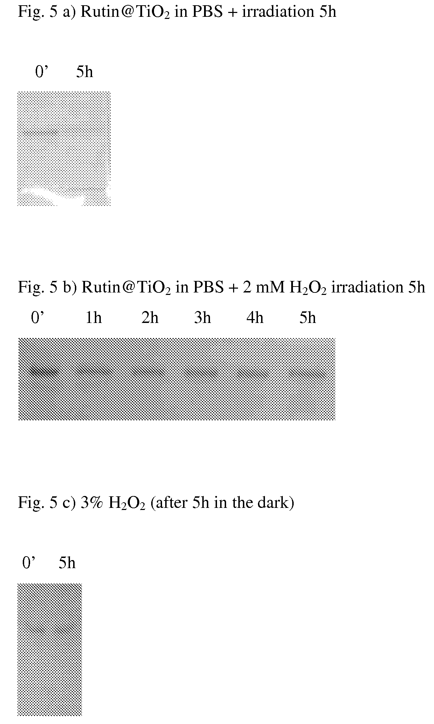

Figure 5a - rutin @ TiO₂ in PBS + irradiation 5 h
Figure 5b - rutin @ TiO₂ in PBS + 2 mM H₂O₂ irradiation 5 h
Figure 5c - 3% H₂O₂ (after 5 h in the dark)

### Example 6. Comparison of activity of the composition according to the invention with composition without active ingredients

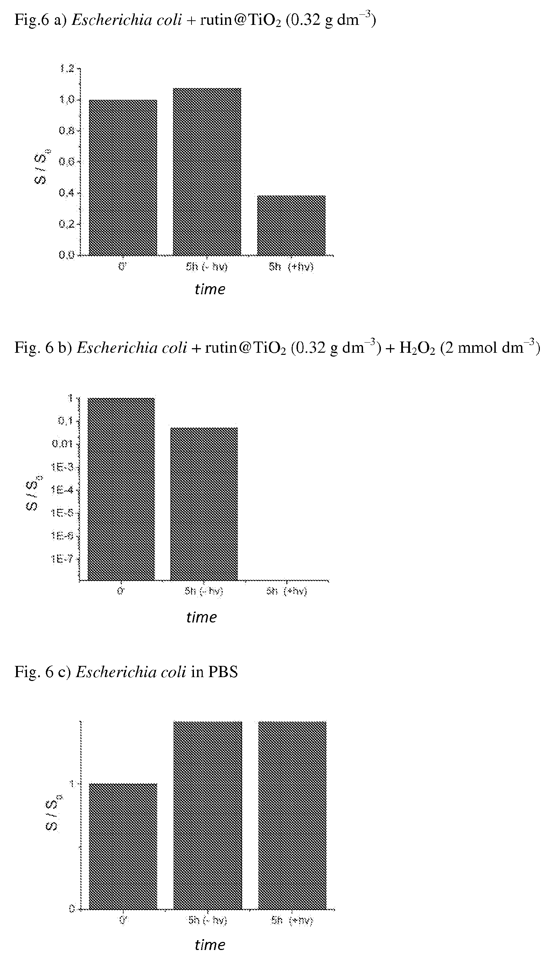

To a suspension of bacterial strain (*Escherichia coli)* of various concentrations in PBS a sterile colloidal solution of rutin @ TiO₂ (filtered through a sterile syringe filter 0.20 µm) was added (Fig. 6a) or a sterile solution of rutin @ TiO₂ with the addition of H₂O₂ (Fig. 6b) to reach its final concentration of 0.32 g dm⁻³ (in terms of TiO₂). Results were compared to the results obtained for a sterile solution of PBS (Fig. 6c).

Tests were performed in chambers of 2 ml capacity using LEDs as the light source (λₘₐₓ = 470 nm, power of 30 mW). Suspensions were irradiated for 5 h at ambient temperature. Small volumes of samples were collected to determine the ability of bacteria to form colonies after irradiation. Analogous control experiments were done in the dark. In this case chambers were incubated for 5h in dark at ambient temperature instead of their exposition to irradiation. Colony forming ability was determined by plating the 100 µl of serial dilutions of bacterial suspension to the minimum medium (Difco) and counting bacterial colonies after 24 h of bacterial growth on the solid medium at 37°C. Results were expressed as survival fraction *S*/*S₀.* The results prove very high activity of tested compositions towards photoinactivation of microorganisms (Fig. 6). No significant cytotoxicity of tested materials to bacterial strains was observed in the dark.

### Example 7. Synergy of the photocatalyst and hydrogen peroxide

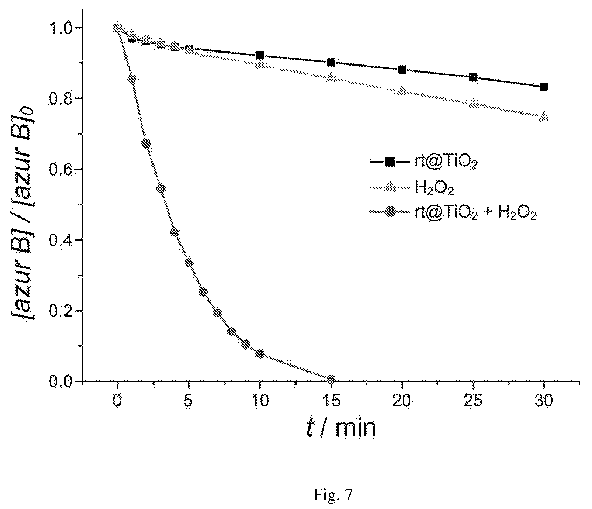

The study was performed using a model system in which the decomposition of the dye azur B was monitored. The results are presented in Figure 7. Photodegradation of azur B was observed in the presence of the photocatalyst rutin @ TiO₂ at the concentration of 0.32 g dm⁻³ (squares), hydrogen peroxide at the concentration of 5 mmol dm⁻³ (triangles) and the mixture of the photocatalyst and hydrogen peroxide at the above mentioned concentrations (circles). The experiment was conducted under the following conditions: initial concentration of azur B was 0.8×10⁻⁴ mol dm⁻³, irradiation with the high-pressure mercury lamp HBO-500 equipped with the 420 nm cut-off filter (exposure 420 < λ < 800 nm).

Surprisingly, the activity of the mixture of the photocatalyst and hydrogen peroxide is considerably strengthened as compared to the separate use of the photocatalyst and hydrogen peroxide.

## Claims

1. The composition in the form of liquid containing colloidal solution, with surface-modified titanium dioxide nanoparticles smaller than 100 nm as a dispersed phase, and the solution of hydrogen peroxide as the dispersing medium, wherein said titanium dioxide nanoparticles are surface-modified with an organic compound, **characterized in that** it exhibits stability in aqueous solution with a pH of 7, and the organic compound is a compound selected from the group consisting of: disodium salt of 4,5-dihydroxybenzene-1,3-disulfonic acid, rutin or ascorbic acid.

2. Composition according to the claim 1, **characterized in that** it shows visible light absorption in the wavelength range (λ) of not less than approximately 400 nm to about 600 nm, preferably up to about 700 nm, and ultraviolet light absorption (λ < 400 nm).

3. Composition according to the claim 1, **characterized in that** it additionally contains a buffer system, preferably isotonic, to maintain the pH in a pharmaceutically acceptable range.

4. Composition according to the claim 3, **characterized in that** it contains surface-modified nanocrystalline titanium dioxide within the concentration range of 0.02 g dm⁻³ to 1 g dm⁻³, hydrogen peroxide, preferably in the amount within the range of 0.001 g dm⁻³ to 0.1 g dm⁻³, and a stabilizer of hydrogen peroxide, preferably EDTA.

5. Composition according to the claims 3 or 4, **characterized in that** it contains additionally an additive showing bacteriocidal and/or mycocidal activity.

## Patentansprüche

1. Zusammensetzung in Form einer Flüssigkeit, die eine kolloidale Lösung enthält, mit oberflächenmodifizierten Titandioxid-Nanopartikeln, die kleiner als 100 nm sind, als dispergierte Phase und Wasserstoffperoxidlösung als Dispersionsmedium, wobei die Titandioxid-Nanopartikel mit einer organischen Verbindung oberflächenmodifiziert sind, **dadurch gekennzeichnet, dass** sie in wässriger Lösung mit einem pH-Wert von 7 Stabilität aufweist und dass die organische Verbindung eine Verbindung ist, die ausgewählt ist aus der Gruppe bestehend aus: Dinatriumsalz von 4,5-Dihydroxybenzol-1,3-disulfonsäure, Rutin oder Ascorbinsäure.

2. Zusammensetzung nach Anspruch 1, **dadurch gekennzeichnet, dass** sie Absorption von sichtbarem icht im Wellenlängenbereich (λ) von nicht weniger als etwa 400 nm bis etwa 600 nm, bevorzugt bis etwa 700 nm, und Absorption von ultraviolettem Licht (λ < 400 nm) zeigt.

3. Zusammensetzung nach Anspruch 1, **dadurch gekennzeichnet, dass** sie zusätzlich ein Puffersystem, bevorzugt ein isotonisches Puffersystem, enthält, um den pH-Wert in einem pharmazeutisch verträglichen Bereich zu halten.

4. Zusammensetzung nach Anspruch 3, **dadurch gekennzeichnet, dass** sie oberflächenmodifiziertes nanokristallines Titandioxid innerhalb des Konzentrationsbereichs von 0,02 g/dm⁻³ bis 1 g/dm⁻³, Wasserstoffperoxid bevorzugt in einer Menge innerhalb des Bereichs von 0,001 g/dm⁻³ bis 0,1 g/dm⁻³ und einen Stabilisator von Wasserstoffperoxid, bevorzugt EDTA, enthält.

5. Zusammensetzung nach den Ansprüchen 3 oder 4, **dadurch gekennzeichnet, dass** sie zusätzlich einen Zusatzstoff enthält, der bakterizide und/oder antimykotische Aktivität aufweist.

## Revendications

1. Composition sous la forme d'une solution colloïdale contenant un liquide, avec des nanoparticules de dioxyde de titane à surface modifiée inférieures à 100 nm en tant que phase dispersée, et la solution de peroxyde d'hydrogène en tant que milieu de dispersion, dans laquelle lesdites nanoparticules de dioxyde de titane ont une surface modifiée avec un composé organique, **caractérisée en ce qu'**elle présente une stabilité dans la solution aqueuse avec un pH de 7 et le composé organique est un composé sélectionné dans le groupe constitué par : le sel disodique de l'acide 4,5-dihydroxybenzène-1,3-disulfonique, la rutine ou l'acide ascorbique.

2. Composition selon la revendication 1, **caractérisée en ce qu'**elle présente une absorption de la lumière visible dans la plage de longueurs d'onde (λ) qui n'est pas inférieure à approximativement 400 nm à environ 600 nm, de préférence jusqu'à environ 700 nm, et une absorption de la lumière ultraviolette (λ < 400 nm).

3. Composition selon la revendication 1, **caractérisée en ce qu'**elle contient en outre un système tampon, de préférence isotonique, pour maintenir le pH dans une plage pharmaceutiquement acceptable.

4. Composition selon la revendication 3, **caractérisée en ce qu'**elle contient du dioxyde de titane nanocristallin à surface modifiée au sein de la plage de concentrations de 0,02 g dm⁻³ à 1 g dm⁻³, du peroxyde d'hydrogène, de préférence dans la quantité au sein de la plage de 0,001 g dm⁻³ à 0,1 g dm⁻³, et un stabilisateur de peroxyde d'hydrogène, de préférence l'EDTA.

5. Composition selon les revendications 3 ou 4, **caractérisée en ce qu'**elle contient en outre un additif présentant une activité bactéricide et/ou mycocide.
